**Europäisches Patentamt**

(19) **European Patent Office**

**Office européen des brevets**

(11) Publication number: **0 113 331**

**A1**

(12) **EUROPEAN PATENT APPLICATION**

(21) Application number: **83870142.3**

(22) Date of filing: **28.12.83**

(51) Int. Cl.³: **C 07 C 121/18**
//C07C120/14

(30) Priority: **30.12.82 US 454748**

(43) Date of publication of application:
**11.07.84 Bulletin 84/28**

(84) Designated Contracting States:
**DE FR GB NL**

(71) Applicant: **Monsanto Company**
**Patent Department 800 North Lindbergh Boulevard**
**St. Louis Missouri 63167(US)**

(72) Inventor: **Hartnett, Robert Lee**
**2027 Bay Street**
**Texas City Texas 77590(US)**

(74) Representative: **McLean, Peter et al,**
**Monsanto Europe S.A. Patent Department Avenue de**
**Tervuren 270-272 Letter Box No 1**
**B-1150 Brussels(BE)**

(54) **Method for the purification of acetonitrile by low temperature phase separation.**

(57) Acetonitrile is recovered from crude aqueous acetonitrile containing streams by cooling the stream until two liquid phases are formed, an aqueous phase containing a predominant amount of water and an organic phase containing a predominant amount of acetonitrile, separating the two phases, and recovering the acetonitrile from the organic phase.

EP 0 113 331 A1

Croydon Printing Company Ltd.

## METHOD FOR THE PURIFICATION OF ACETONITRILE
## BY LOW TEMPERATURE PHASE SEPARATION

### BACKGROUND OF THE INVENTION

This invention relates to a process for separation and recovery of substantially pure acetonitrile from crude waste organic streams containing acetonitrile, water and one or more additional organic components. More particularly, the invention is a low temperature phase separation process for recovery of acetonitrile.

Acetonitrile is produced as a by-product in the production of acrylonitrile from propylene, oxygen and ammonia in the presence of an ammoxidation catalyst. The crude acetonitrile stream, after separation from the acrylonitrile product, usually contains a mixture of water, acetonitrile, hydrogen cyanide and often lesser amounts of other organic components such as acrylonitrile, acrolein, acetone, propionitrile, benzonitrile, acetaldehyde, various cyanohydrins and the like.

Conventional methods for separation of acetonitrile from such mixtures have typically involved either distillation or solvent extraction. In U.S. 3,451,899 to Sheely, multiple distillation columns or stages are shown wherein the hydrogen cyanide is removed first and the acetonitrile separated in one or more additional distillation steps from the remaining components in the crude organic stream. Such methods are costly and consume large amounts of energy since most of the water is removed by distillation. Recovery of pure, water-free acetonitrile from such mixtures by mere distillation is not possible due to the presence of azeotropic mixtures. Organic solvent extraction methods using a variety of organic extractants have also been used to purify acetonitrile. An example of such solvent extraction methods is disclosed in U.S. 3,870,746 to Lussling et al. This patent discloses recovery of pure acetonitrile from aqueous acetonitrile by extraction with an organic solvent, such as a hydrocarbon mixture consisting essentially of alkylbenzenes having boiling points between $187^{o}$ and $213^{o}C.$, which has low miscibility with water, has a boiling point higher than acetonitrile ($81^{o}C.$) and does not form an azeotrope with acetonitrile. Additional distillation steps are required to remove low boiling organic components.

This invention provides an improved method for separation of acetonitrile from water which does not involve extensive distillation or solvent extraction procedures.

## SUMMARY OF THE INVENTION

The invention is a process for the recovery of acetonitrile from a crude aqueous acetonitrile stream comprising cooling the stream until two liquid phases are formed, an aqueous phase containing a predominant amount of water and an organic phase containing a predominant amount of acetonitrile, separating the two phases, and recovering the acetonitrile from the organic phase. Recovery is by distillation or any other suitable means.

In a further aspect of the invention the acetonitrile concentration in the organic phase may be increased by further cooling, decantation and freeze crystallization to remove additional amounts of water prior to distillation.

## DETAILED DESCRIPTION OF THE INVENTION

In the practice of the invention the crude acetonitrile stream resulting from ammoxidation of propylene which is subjected to the recovery process usually comprises a mixture containing acetonitrile, water and lesser amounts of other organic components. Typically, the stream comprises a mixture containing 40 to 60% and in some situations up to 70% acetonitrile by weight, up to 8% hydrogen cyanide and up to 5% by weight of volatile nitriles such as propionitrile and benzonitrile. Smaller amounts of other organic compounds such as acrylonitrile, acetone, acetaldehyde, acrolein and various cyanohydrins plus water make up the balance. Acetonitrile forms azeotropic mixtures which makes separation and recovery of pure, essentially water-free acetonitrile from such mixtures virtually impossible.

It has been found that by carefully cooling this crude acetonitrile stream a phase separation occurs well above the initial freezing point of the mixture. The exact freezing point is dependent upon the composition of the mixture. Phase separation into an organic phase enriched in acetonitrile and an aqueous phase enriched in water will

0113331

occur at any point between about 6 and $-13^{\circ}$C. Significant enrichment of the acetonitrile in the organic phase occurs reaching greater than 70% by weight acetonitrile at $-9^{\circ}$C. Mixtures of pure acetonitrile and water do not begin to separate until the temperature is lowered to $-3^{\circ}$C. The addition of either acrylonitrile or hydrogen cyanide in the concentrations normally found in crude acetonitrile to the acetonitrile/ water binary raise the temperature at which separation starts with cyanide having a much greater effect on phase separation than acrylonitrile.

It is desirable and preferable to obtain the highest possible concentration of acetonitrile in the organic phase. Up to 85% by weight acetonitrile can be concentrated in the organic phase. It is possible to achieve 90% or more acetonitrile in the organic phase using a freeze crystallization step to remove additional amounts of water which further reduces the amount of distillation required for final separation of the acetonitrile product.

The invention is further illustrated by the following examples showing specific and preferred embodiments of the process.

## EXAMPLE 1

A series of five separate charges of 44.8 grams (50 ml) each and consisting of a mixture of 50% by weight of acetonitrile and 50% by weight of water were prepared. The charges were cooled to the indicated temperatures and the phases analyzed by gas chromatography to determine the % acetonitrile (ACN) in each charge upon phase separation. The results are shown in Table 1 below.

## TABLE 1. PHASE SEPARATION

### Acetonitrile/Water
### Binary

| Charge No. | Temperature °C | Aqueous Phase ACN (Wt. %) | Organic Phase ACN (Wt. %) |
|---|---|---|---|
| 1 | -3 | 43.57 | 72.34 |
| 2 | -4 | 42.24 | 75.16 |
| 3 | -6 | 38.31 | 76.99 |
| 4 | -7 | 36.19 | 80.2 |
| 5 | -9 | 35.47 | 82.48 |

This example shows that acetonitrile may be concentrated to greater than 80% by weight in the organic phase at $-7^\circ$ to $-9^\circ$C. At $-14^\circ$C. the water begins to form ice in the organic phase.

### EXAMPLE 2

In this example six charges of 43.61 grams (50 ml) each were prepared. Each charge consisted of a mixture of 56.9% by weight of acetonitrile, 41.94% by weight of water and 1.16% by weight of acrylonitrile. The charges were cooled to the indicated temperature for each one and the phases were analyzed by gas chromatography to determine the % acetonitrile in each phase for the six charges upon phase separation. The results are reported in Table 2 below.

## TABLE 2. PHASE SEPARATION

### Acetonitrile/Acrylonitrile/Water

| Charge No. | Temperature °C | Aqueous Phase ACN (Wt. %) | Organic Phase ACN (Wt. %) |
|---|---|---|---|
| 1 | 3 | -- | 60.09 |
| 2 | 2 | 30.90 | 61.12 |
| 3 | 0 | 27.99 | 63.52 |
| 4 | -3 | 25.16 | 67.64 |
| 5 | -6 | 24.42 | 70.62 |
| 6 | -9 | 25.30 | 71.00 |

## EXAMPLE 3

Following the procedure of Example 1, seven 50 ml charges were prepared consisting of 57.02% by weight of acetonitrile, 37.13% by weight of water and 5.85% by weight of hydrogen cyanide. The results are reported in Table 3 below.

### TABLE 3. PHASE SEPARATION

Acetonitrile/Hydrogen Cyanide/Water

| Charge No. | Temperature °C | Aqueous Phase ACN (Wt. %) | Organic Phase ACN (Wt. %) |
|---|---|---|---|
| 1 | 6 | 50.15 | 64.52 |
| 2 | 5 | 41.14 | 70.23 |
| 3 | 3 | 40.07 | 71.19 |
| 4 | 0 | 37.14 | 75.12 |
| 5 | -3 | 33.79 | 78.65 |
| 6 | -6 | 31.43 | 79.24 |
| 7 | -9 | 28.81 | 81.41 |

Normally, mixtures of water and acetonitrile are miscible at room temperatures. Using the Clausins-Clapeyron Equation one would predict that a 50-50 mixtures of acetonitrile and water would freeze at -53°C without any prior phase separation. The Equation cannot predict phase separation and, in fact, no phase separation occurs for mixtures of water and acetic acid, water and methanol or water and hydrogen cyanide in any proportions prior to the point at which the mixture freezes. These examples thus demonstrate that surprisingly phase separation does occur prior to freezing for water-acetonitrile mixtures within the indicated temperature ranges.

An additional advantageous feature of the process is that distillation problems associated with water-acetonitrile azeotropies are avoided since the phase separation process removes most of the water from the water-acetonitrile mixture prior to the final distillation step. The organic phase contains a predominant amount of acetonitrile, i.e., greater than 70% by weight and up to about 85% by weight. The water is concentrated predominately in the aqueous phase in amounts ranging from about 50% by weight to 75% or more by weight.

It is understood that the specific embodiments described in the examples are for purposes of illustration only and are not intended to limit the invention, which is obviously subject to variations and modifications without departing from the spirit and scope thereof as defined in the claims.

CLAIMS:

1.      A process for the recovery of acetonitrile from a crude aqueous acetonitrile stream comprising cooling the stream until two liquid phases are formed, an aqueous phase containing a predominant amount of water and an organic phase containing a predominant amount of acetonitrile, separating the two phases, and recovering the acetonitrile from the organic phase.

2.      The process of Claim 1 wherein the stream is cooled to a temperature of from about 6 to $-13^{\circ}C$.

3.      The process of Claim 1 wherein the organic phase contains at least 70 wt. % acetonitrile.

4.      The process of Claim 1 wherein the organic phase contains at least 85 Wt. % acetonitrile.

5.      The process of Claim 1 wherein the organic phase contains hydrogen cyanide.

6.      The process of Claim 1 wherein the organic phase contains acrylonitrile.

7.      The process of Claim 1 wherein the acetonitrile is recovered from the organic phase by distillation or solvent extraction.

8.      The process of Claim 1 wherein the stream comprises a mixture of water, acetonitrile and hydrogen cyanide.

9.      The process of Claim 1 wherein the stream comprises a mixture of water, acetonitrile and acrylonitrile.

10.      The process of Claim 1 wherein the stream comprises a mixture of water, acetonitrile, hydrogen cyanide and acrylonitrile.

**EUROPEAN SEARCH REPORT**

## DOCUMENTS CONSIDERED TO BE RELEVANT

EP 83870142.3

| Category | Citation of document with indication, where appropriate, of relevant passages | Relevant to claim | CLASSIFICATION OF THE APPLICATION (Int. Cl. ³) |
|---|---|---|---|
| X | US - A - 2 417 862 (C.H. DALE)<br>* Claims *<br>-- | 1-4,7 | C 07 C 121/18/<br>C 07 C 120/14 |
| A | DE - A1 - 2 629 189 (MONSANTO CO.)<br>* Claim 2 *<br>-- | 1 | |
| D,A | US - A - 3 451 899 (H.R. SHEELY)<br>* Example *<br>---- | 1,10 | |

|  |
|---|
| **TECHNICAL FIELDS SEARCHED (Int. Cl. ³)** |
| C 07 C 121/00<br>C 07 C 120/00 |

The present search report has been drawn up for all claims

| Place of search | Date of completion of the search | Examiner |
|---|---|---|
| VIENNA | 07-03-1984 | HOFBAUER |